# DEMANDE DE BREVET EUROPEEN

(11) **EP 0 582 849 A1**
(43) Date de publication de la demande: **16.02.1994**
(21) Numéro de dépôt: 93111245.2
(22) Date de dépôt: 13.07.1993
(51) Int. Cl.: A61M 5/32, A61M 5/50

(54) **Seringue**

(30) Priorité: 12.08.1992 CH 2527/92; 28.01.1993 CH 242/93
(71) Demandeur: Somers, Brice, CH-1253 Vandoeuvres (CH); Hauf, Eric, CH-1005 Lausanne (CH)
(72) Inventeur: Somers, Brice, CH-1253 Vandoeuvres (CH); Hauf, Eric, CH-1005 Lausanne (CH)
(74) Mandataire: Micheli & Cie

(57) **Abrégé**

La seringue comprend un corps cylindrique (1), un piston (11,15) déplaçable linéairement dans le corps (1), un porte-aiguille (28) également déplaçable linéairement dans le corps (1) et par rapport au piston (11,15) et une aiguille (24). L'aiguille (24), avant utilisation de la seringue, est située, hermétiquement protégée, à l'intérieur du cylindre (1), et est montée sur le porte-aiguille par l'intermédiaire d'un accouplement du type bouton-pression permettant sa séparation du porte-aiguille. Le corps (1) de la seringue est obturé à son extrémité distale par une pièce de fermeture (4) comportant un opercule élastomère (9) perforable par l'aiguille. Le porte-aiguille (28) et la pièce de fermeture distale (4) comportent des formations d'accouplement (31,10) par encliquetage non déverrouillables une fois encliquetées. L'extrémité arrière de l'aiguille (24) et le piston (11, 15) comportent des formations d'accouplement par encliquetage (20,26) non déverrouillables une fois encliquetées. La distance minimale entre le piston (11,15) et le porte-aiguille (28), est déterminée par un élément (29) dont la résistance à la compression est plus faible à l'état humide ou mouillé qu'à l'état sec.

## Description

La présente invention a pour objet une seringue à usage médical, notamment une seringue d'une totale sécurité d'emploi tant pour le personnel soignant, hospitalier ou non, que pour les patients, même les patients procédant eux-mêmes à leurs injections. La seringue selon l'invention a donc pour but une utilisation de toute sécurité pour éviter notamment les risques de contamination tant du personnel soignant que des utilisateurs ou patients, facile à utiliser et à emploi unique. De plus, cette nouvelle seringue est d'un faible coût de revient.

Dans ce but, l'invention consiste en une seringue munie d'une aiguille qui n'est pas apparente avant l'emploi et se trouve après l'emploi de nouveau enfermée dans le cylindre de la seringue, supprimant ainsi toute manipulation manuelle de l'aiguille, et donc tout risque de contamination.

Cette seringue, non réutilisable, peut ainsi être jetée après emploi, sans précautions particulières.

Les seringues traditionnelles, bon marché et d'un maniement aisé, sont dangereuses par la possible contamination lors du capuchonnage de l'aiguille (piqûre de l'utilisateur, par exemple) ou toute autre manipulation ultérieure (débarras par exemple).

Face aux maladies transmissibles par le sang, telles que SIDA, hépatite virale, syphilis, etc. de nouvelles seringues sont apparues tendant à réduire les risques de contamination et de réutilisation. Ces seringues sont plus coûteuses et d'une manipulation plus compliquée que les seringues traditionnelles et le danger de contamination par une aiguille souillée n'est pas toujours exclu.

On connait entre autre des seringues du type VACUTAINER fabriquées aux USA, VENOJECT fabriquées au Japon ou VACUETTE fabriquées en Hollande. Tous ces systèmes ne permettent que le prélèvement sanguin et nécessitent l'introduction préalable d'un collecteur à aiguille dans la veine du patient. Cette aiguille étant en place, on y connecte des récipients ou tubes mis sous vide qui se remplissent alors de sang.

De tels systèmes nécessitent une manipulation de l'aiguille elle-même par l'opérateur tant pour son introduction dans une veine, pour sa connexion au tube que pour la jeter après l'emploi. Toutes ces manipulations sont répétitives et de plus l'évacuation des aiguilles usagées nécessite des précautions particulières, emballages, capuchons, etc.

De plus, un tel système ne permet pas de procéder à des injections, mais seulement à des prises de sang.

Un autre inconvénient de ce système réside dans le fait que le sang ne peut être prélevé que dans des tubes mis préalablement sous vide. Cela nécessite un prélèvement ultérieur du sang à l'aide d'une seringue traditionnelle lorsqu'on veut faire des hémocultures pour le transférer dans des récipients contenant un milieu de culture, rendant ainsi dérisoire le système sophistiqué utilisé dans le but de prévenir toute contamination.

Lorsque plusieurs tests doivent être effectués, cela implique de répéter l'opération de prélèvement du sang à l'aide d'un tube sous vide. Ces manipulations répétées sont désagréables pour le patient qui doit supporter la présence continue du collecteur inséré dans une veine. Un autre inconvénient de ces systèmes à tube sous vide réside dans le fait que ces tubes doivent être en verre et sont donc cassables.

Enfin, le prélèvement de sang à l'aide du vacuum du tube peut, sous certaines circonstances particulières, provoquer l'éclatement des globules rouges ou l'obstruction d'une veine par collapse de ses parois puisqu'il est impossible d'ajuster le taux de succion ou du vacuum aux conditions particulières du patient.

Bien que le personnel médical et para-médical se soit habitué à l'emploi des systèmes utilisant les éprouvettes à vide, ils ont néanmoins toujours gardé une préférence pour la simplicité de la seringue traditionnelle. Or, avec l'invention en question, toute la simplicité des seringues traditionnelles est présente, mais sans risques d'infection pour l'utilisateur.

On connaît également le système MONOVETTE fabriqué en Allemagne qui est semblable au système VACUTAINER au fait près que les tubes collecteurs sous vide sont remplacés par des cylindres munis de pistons à l'instar d'une seringue traditionnelle. Les risques de contamination sont ici également grands.

On connaît du document WO 90/06148 une seringue comportant un corps tubulaire à l'intérieur duquel se déplacent linéairement un piston et un porte-aiguille, le piston et le porte-aiguille pouvant être accouplés et désaccouplés. Avant usage, l'aiguille est logée dans le corps tubulaire, elle en est extraite par un déplacement du porte-aiguille sous l'action du piston, contre l'action d'un ressort, et est maintenue en position sortie, opérationnelle, par encliquetage du porte-aiguille sur un épaulement interne du corps. Une fois dans cette position, la seringue peut être utilisée et réutilisée autant de fois que l'on veut. En accouplant le piston au porte-aiguille, il est possible de retirer volontairement celle-ci dans le corps, mais cet escamotage de l'aiguille doit être volontaire et n'est pas automatique. De plus, le système est compliqué et onéreux. Cette seringue ne peut en outre pas être pré-stérilisée sans opération complémentaire puisqu'elle est ouverte à l'avant.

On connaît des documents EP 0 326 983 et EP 0 347 742 des seringues qui permettent également de retirer volontairement à la fin de son utilisation l'aiguille dans le corps de la seringue. Ici également, cette opération est volontaire et non automatique ce qui autorise une utilisation répétée de la seringue. De plus, avant usage, l'aiguille de la seringue est située hors du corps et ne peut donc pas être pré-stérilisée sans protection supplémentaire.

Les mêmes inconvénients se retrouvent dans les seringues décrites dans les documents US 4.507.117 et US 4.675.005, car en effet la seringue ne peut pas être préstérilisée sans protection supplémentaire puisque l'aiguille est hors du corps de la seringue avant usage et nécessite un capuchon et que d'autre part le retrait de l'aiguille dans le corps de la seringue résulte d'une action volontaire, de sorte que la seringue est réutilisable. Cette seringue fait usage d'un mécanisme à ressort métallique et l'aiguille ne peut pas être séparée du porte-aiguille. De plus, pour retirer l'aiguille on doit effectuer une manipulation bi-directionelle provoquant un déplacement axial et rotatif du piston. Enfin, le coût de fabrication est élevé.

La présente invention a pour objet une seringue tendant à obvier aux inconvénients des seringues connues et permettant de résoudre de façon simple, efficace, incontournable et peu coûteuse, notamment les problèmes suivants :
1. Avant emploi, la seringue et son aiguille doivent être pré-stérilisables sans devoir être obligatoirement enfermée dans un
   emballage.
2. La seringue doit avant emploi avoir son aiguille enfermée hermétiquement à l'intérieur du corps.
3. La seringue doit pouvoir être utilisée tant pour des prises de sang que pour des injections de produits.
4. Après un seul et unique usage de la seringue, l'aiguille doit, après retrait par un mouvement linéaire seulement dans le corps de la seringue, ne plus pouvoir en être extraite, ce qui prévient toute contamination ultérieure et garantit un usage unique.

La seringue à usage médical non réutilisable selon l'invention obviant aux inconvénients précités et permettant de résoudre les problèmes énumérés ci-dessus se distingue par les caractéristiques énumérées à la revendication 1.

Les dessins annexés illustrent schématiquement et à titre d'exemple des formes d'exécution de la seringue selon l'invention.

La figure 1 illustre une seringue selon l'invention avant emploi, sa tige étant désaccouplée.

La figure 2 illustre la seringue prête à l'emploi.

La figure 3 illustre le prélèvement ou aspiration de liquide ou de sang à l'aide de la seringue.

La figure 4 illustre l'état de la seringue après expulsion du sang prélevé dans une éprouvette ou autre récipient, ou après injection du liquide dans le corps.

La figure 5 illustre la seringue après usage l'aiguille étant à nouveau enfermée dans le corps et inclinée.

La figure 6 est un détail du crochet du piston.

La figure 7 est une vue de dessus de la partie arrière de l'aiguille.

La figure 8 est une vue de dessus du porte-aiguille.

La figure 9 est une vue similaire à la figure 1 d'une seconde forme d'exécution de la seringue.

La figure 10 est une vue similaire à la figure 4 de la seconde forme d'exécution de la seringue.

Les figures 11 à 20 illustrent une troisième forme d'exécution de la seringue.

Les figures 21 à 26 illustrent une quatrième forme d'exécution de la seringue.

Les figures 27 et 28 illustrent une variante de cette quatrième forme d'exécution de la seringue.

La figure 29 illustre une variante de la fixation de l'élément déformable sur le piston.

La présente invention a pour objet une seringue à usage médical non réutilisable, pouvant servir soit pour le prélèvement de liquide, généralement du sang, soit pour l'injection d'une solution médicamenteuse ou autre. Comme on le verra de la description détaillée qui suit, cette seringue présente de très nombreux avantages par rapport aux seringues existantes, ainsi que par rapport à celles, pour la plupart non commercialisées, qui sont décrites dans les documents antérieurs discutés dans l'introduction. Ces principaux avantages sont notamment :
1. L'aiguille fait partie intégrante de la seringue et ne peut pas être séparée de celle-ci évitant toute manipulation directe de cette aiguille, telles que fixation, capuchonnage, rangement, etc.
2. Avant emploi, l'aiguille est protégée à l'intérieur du cylindre qui est fermé hermétiquement. De ce fait, l'ensemble de la seringue peut être stérilisé après fabrication et montage, par exemple par des rayonnements ionisants, et ne nécessite pas d'emballage particulier.
3. En fin d'utilisation, l'aiguille, éventuellement désolidarisée du porte-aiguille, est prise par le piston ce qui permet sa rétractation automatique à l'intérieur du cylindre par le mouvement linéaire du piston. Ainsi, non seulement l'utilisateur est protégé contre toute contamination ou infection, mais toute surface à l'extérieur de la seringue n'étant pas souillée, elle peut être simplement jetée ou mise au rebut sans précaution particulière, toute pièce contaminée ou souillée étant hermétiquement contenue dans le cylindre. Un usage vraiment unique de la seringue est assuré, l'aiguille étant automatiquement biaisée une fois rétractée dans le cylindre et ne pouvant plus, même volontairement, être ressortie.
4. En fin d'utilisation, le piston est définitivement solidaire de l'aiguille ce qui interdit toute réutilisation de la seringue.
5. Un cône à l'extrémité du cylindre empêche tout risque de contamination par une éventuelle souillure de sang restée sur la surface extérieure du diaphragme élastomérique.
6. En fin d'utilisation, la tige du piston peut être cassée à l'extérieur de la seringue pour en réduire l'encombrement offrant ainsi une autre garantie de non réutilisation.
7. L'utilisation de la seringue est extrêmement simple, elle consiste uniquement en un mouvement de va et vient rectiligne du piston dans le cylindre à l'aide d'une tige et ceci sans rotation, vissage, dévissage ou positionnement angulaire d'une pièce par rapport à une autre pour actionner un accouplement.
8. Une fois utilisée, la seringue peut être jetée sans précautions spéciales, sa surface extérieure n'est pas contaminée, toute souillure restant hermétiquement à l'intérieur.
9. Enfin, la construction de la seringue est simple faisant appel à des techniques bien connues et peu onéreuses. Elle est entièrement réalisée en matières plastiques extrudées, moulées ou injectées, à l'exception de l'aiguille ou d'un éventuel ressort bien entendu, ce qui permet une fabrication en grande série, à haute cadence et à bas prix. Son montage est simple, se prête à l'automation et ne fait pas appel à de la main d'oeuvre qualifiée.
   Sa construction et son montage sont simples, généralement sans ressorts, et sans mécanisme compliqué, peu coûteux et appropriés pour une production industrielle.

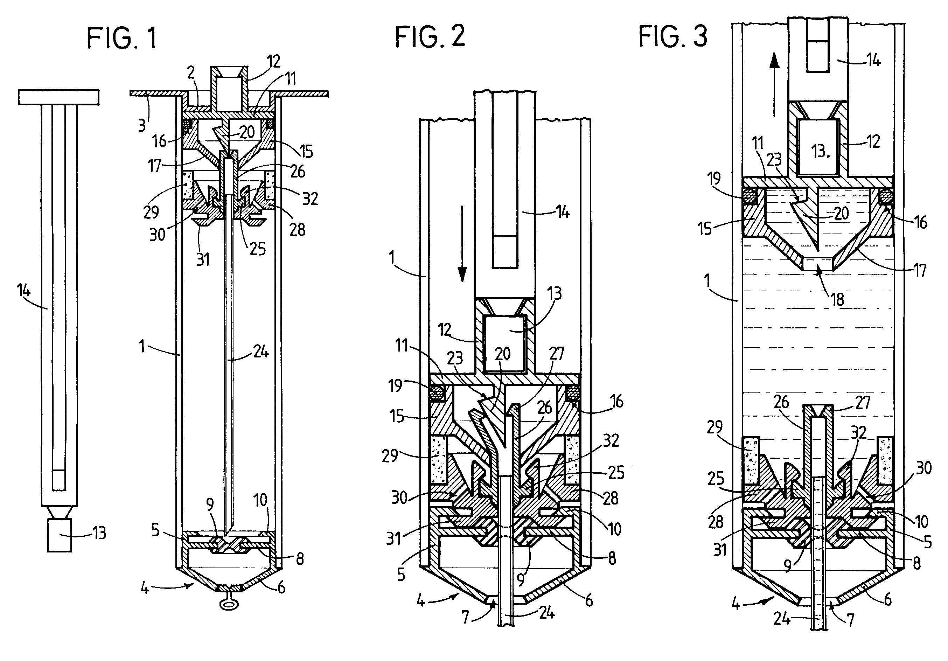

En référence aux dessins, la première forme d'exécution de la seringue illustrée aux figures 1 à 5 est constituée par un tube cylindrique 1 obtenu par extrusion et réalisé par exemple en polycarbonate ou polypropylène transparent. Ce tube extrudé est sectionné à la longueur désirée dépendant de la capacité de la seringue, de sa commodité d'utilisation et d'autres facteurs habituels.

Ce cylindre 1 de la seringue est obturé à son extrémité proximale par un couvercle également en matière plastique comportant une partie cylindrique centrale 2 s'emboîtant exactement dans le cylindre 1 et une collerette 3 de plus grand diamètre. En position assemblée, le couvercle 2,3 est soudé, collé ou fixé de toute autre manière de façon étanche sur cette extrémité proximale du cylindre 1 de la seringue.

L'autre extrémité, l'extrémité distale du cylindre 1 est obturée par une pièce de fermeture 4 moulée ou injectée en résine thermoplastique, généralement du polystyrène. Cette pièce de fermeture 4 comporte une partie cylindrique 5 d'un diamètre externe correspondant au diamètre interne du cylindre 1 et enfilée dans celui-ci. La partie frontale extérieure 6 de cette pièce de fermeture 4 est tronconique et munie d'une ouverture centrale 7.

Cette ouverture 7 peut être obturée, avant l'emploi de la seringue par un bouchon ou alors par une pellicule auto-collante pelable. On peut également obturer cette ouverture par une portion de matière synthétique venue de fabrication avec la paroi 6, mais séparée de celle-ci par une ligne de rupture. Cette portion peut comporter un organe de préhension, par exemple une boucle, permettant d'arracher cette portion d'obturation au moment de l'utilisation de la seringue pour libérer l'ouverture 7 donnant passage à l'aiguille.

Cette pièce de fermeture 4 est également fixée de façon étanche sur le cylindre 1 par collage, soudage ou tout autre moyen adéquat.

Etant donné que les matières constituant le cylindre 1, le couvercle 2,3 et la pièce de fermeture 4 sont compatibles et thermoformables, leur assemblage peut se faire par "spin welding" ou soudure par friction ce qui est très simple et rapide.

La pièce de fermeture 4 de l'extrémité distale du cylindre 1 comporte encore une paroi transversale 8 munie d'une ouverture centrale. Une bague ou opercule 9 en un élastomère est surmoulée sur cette paroi transversale 8 de manière à obturer de façon étanche l'ouverture de cette paroi transversale 8. Cet opercule 9 peut comporter dans sa partie centrale une zone de plus faible épaisseur destinée, comme on le verra plus loin, à être percée par l'aiguille de la seringue. Cette partie frontale centrale de l'opercule 9 comporte une creusure destinée à récupérer toute souillure de la surface externe de l'aiguille lors de sa rétraction dans le cylindre évitant ainsi toute dispersion de cette souillure.

Cette pièce de fermeture distale 4 comporte encore, située à l'intérieur du cylindre 1 de la seringue, une collerette de retenue 10 dont le bord interne est biseauté, la partie de plus faible diamètre de l'ouverture de cette collerette 10 étant située vers l'extrémité distale de la seringue.

La seringue comporte encore un piston formé d'une plaque arrière 11 munie d'une partie femelle 12 d'un accouplement. Cette partie femelle d'accouplement 12 traverse la partie centrale 2 du couvercle 2,3 par un orifice de celui-ci et est accessible de l'extérieur de la seringue. Cette partie femelle d'accouplement 12 est destinée à coopérer avec la partie mâle 13 d'un accouplement correspondant situé à l'extrémité d'une tige permettant l'actionnement du piston 14.

Le piston comporte encore une jupe 15 cylindrique dans sa partie supérieure, d'un diamètre correspondant au diamètre interne du cylindre 1 et comportant un épaulement 16. La partie frontale 17 de cette jupe est tronconique et est munie d'un orifice central 18. La plaque 11 munie de la partie d'accouplement 12 et la jupe 15,16,17 sont fixées rigidement l'une à l'autre par soudage, collage, etc. Ces parties sont réalisées en matière thermoplastique telle que du polyacétal et obtenues par moulage ou injection. Un joint "O ring" 19 est disposé dans la gorge ménagée entre l'épaulement 16 de la jupe et la plaque 11 assurant ainsi une étanchéité parfaite entre ce piston 11-19 et le cylindre 1.

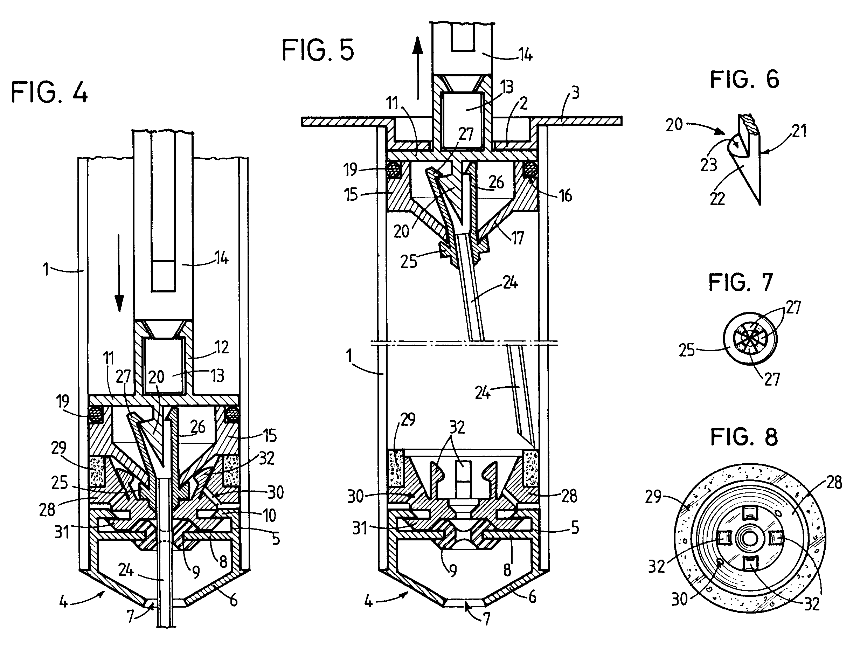

Ce piston comporte encore un crochet 20 solidaire de la plaque 11 et s'étendant à l'intérieur de la jupe 15, 17. Ce crochet présente dans l'exécution illustrée (figure 6) une paroi plane 21 située dans l'axe du cylindre 1 et une formation de retenue constituée par un demi-cône 22 dont le diamètre va en s'élargissant en direction de la plaque 11, demi-cône terminé par une face plane 23 légèrement inclinée par rapport à la plaque 11.

Cette seringue comporte encore bien entendu une aiguille creuse 24 en acier, biseautée à son extrémité distale et munie à son extrémité proximale d'un surmoulage en matière thermoplastique, par exemple en polyacétal comprenant d'une part un disque 25 dont la face supérieure est creusée de manière à ce que son bord externe constitue une formation de retenue, comme on le verra plus loin et, d'autre part des lamelles 26, quatre dans l'exemple illustré, munies de crochets 27 à leurs extrémités. Ces lamelles 26 flexibles s'étendent en position normale dans l'axe de l'aiguille et sont disposées à l'intérieur d'une circonférence dont le diamètre correspond à celui de l'orifice central 18 du piston de manière à pouvoir pénétrer dans l'espace interne de la jupe 15,17 de celui-ci. La longueur de ces lamelles 26 est suffisante pour que lorsqu'elles sont introduites à fond dans la jupe 15, 17 du piston, au moins un des crochets 27 vienne se crocher sur la face plane 23 du crochet 20 du piston pour solidariser définitivement l'aiguille 24 de ce piston 11,22.

L'aiguille 24 et son surmoulage 25,26,27 sont normalement montés sur un porte-aiguille coulissant librement dans le cylindre 1. Ce porte-aiguille comporte un corps rigide 28 d'un diamètre correspondant au diamètre interne du cylindre 1 muni en direction de l'extrémité proximale de la seringue d'un évidement annulaire recevant une bague 29. Le corps 28 est en matière synthétique moulable ou injectée telle que du polyacétal. Le corps 28 comporte encore au moins un passage ou évent 30 créant une liaison entre les parties de la cavité du cylindre 1 situées de part et d'autre de ce porte-aiguille de façon à ce que celui-ci puisse coulisser librement dans le cylindre 1.

Ce porte-aiguille mobile comporte encore des premières formations d'encliquetage 31 sur sa face distale constituée par des pattes ou ergots présentant une face biseautée et qui lorsqu'ils sont appliqués contre la collerette 10 de la pièce de fermeture distale se déforment élastiquement, puis s'encliquettent sur cette collerette 10 constituant une liaison permanente irréversible entre le porte-aiguille et la pièce de fermeture distale.

Le porte-aiguille comporte encore une seconde formation d'encliquetage 32 émergeant du corps 28 en direction de l'extrémité proximale de la seringue et coopérant avec le rebord du disque 25 du surmoulage de l'aiguille. Ces formations d'encliquetage 32 sont constituées par des languettes déformables élastiquement et munies à leurs extrémités de bossages coopérant avec le rebord du disque 25. L'accouplement ainsi réalisé est réversible, comme on le verra plus loin l'aiguille et son surmoulage pouvant être séparés du porte-aiguille lors du fonctionnement de la seringue.

En effet, il faut que le surmoulage 25 de l'aiguille soit accouplé au porte-aiguille 32 avec une force suffisante pour éviter tout désaccouplement de ces éléments lors de l'introduction de l'aiguille dans la veine du patient ou dans un muscle de celui-ci. Par contre, la force de cet accouplement doit permettre la séparation de l'aiguille du porte-aiguille lorsque l'aiguille est accouplée au piston et que celui-ci est rétracté.

La bague 29 placée dans l'évidement annulaire du corps 28 du porte-aiguille est réalisée en une matière naturelle ou synthétique rigide à l'état sec mais susceptible de ramollir au contact d'un liquide qu'elle absorbe.

Lorsque la seringue est neuve, la bague 29 est sèche et dure, elle présente donc une résistance appréciable à la compression. La hauteur de cette bague 29 est telle que lorsque le piston est déplacé en direction du porte-aiguille, la tranche supérieure de cette bague 29 bute contre la face inférieure de la partie cylindrique 15 de la jupe du piston et que, la bague 29 résistant, les crochets 27 des lamelles 26 du surmoulage de l'aiguille 24 n'arrivent pas à remonter suffisamment sur le crochet 20 du piston pour s'accrocher sur la face 23 de celui-ci. Ainsi lorsque la bague 29 est sèche et dure ou rigide, il n'est pas possible d'encliqueter l'embout 25,26 de l'aiguille 24 sur le crochet 20 du piston. Comme on le verra plus loin, cet encliquetage est possible lorsque la bague 29 aura été ramollie par un liquide, car la matière dont elle est réalisée perd sa rigidité, sa dureté et sa résistance à la compression.

Comme on l'a vu de cette description détaillée de la seringue, celle-ci comporte un cylindre 1, obturé à chacune de ses extrémités avant son usage et trois ensembles contenus à l'intérieur du cylindre, le piston 15, l'aiguille 24-25 et le porte-aiguille 31-32, tous trois déplaçables par rapport au cylindre et les uns par rapport aux autres. De plus, le porte-aiguille 31 est accouplable de façon irréversible, non désaccouplable, avec l'extrémité distale du cylindre 10; l'aiguille peut être accouplée de façon irréversible, non découplable, avec le piston et l'aiguille est accouplée de façon découplable avec le porte-aiguille.

Enfin, cette seringue comporte un élément, situé entre le piston et le porte-aiguille, dont la dureté et donc la résistance à la compression diminue lorsqu'il a été exposé à un liquide, cet élément n'autorisant l'accouplement de l'aiguille sur le piston que lorsque sa résistance a diminué consécutivement à son exposition à un liquide.

Le fonctionnement de la seringue décrite est le suivant :
1. L'usager prend une seringue stérile, neuve, telle qu'illustrée à la figure 1, si la tige ne fait pas partie intégrante du piston il accouple la tige 14 sur le piston 11-23 de la seringue à l'aide de l'accouplement à l'encliquetage 12,13 et dégage l'ouverture 7.
2. En appuyant sur la tige 14 et en maintenant le corps de la seringue par la collerette 3, l'usager déplace le piston 11-23 en direction de l'extrémité distale de la seringue. Ce faisant, la partie cylindrique 15 de la jupe du cylindre vient s'appuyer sur la bague 29, rigide et résistante puisque sèche, du porte-aiguille 28-32 et entraîne de ce fait ce porte-aiguille, ainsi que l'aiguille 24-26 qui lui est accouplée. Ce faisant l'extrémité biseautée et aiguisée de l'aiguille 24 perce l'opercule en élastomère 9, l'aiguille sort du cylindre 1.
   L'usager continue de déplacer le piston vers l'extrémité distale de la seringue jusqu'à ce que le porte-aiguille soit encliqueté, et donc relié rigidement et de façon non démontable, sur la pièce de fermeture distale 4 par le crochetage des ergots 31 venant se placer sous la collerette 10 (voir figure 2). Pendant ce déplacement du piston, du porte-aiguille et de l'aiguille, l'air contenu dans le cylindre traverse le porte-aiguille par les passages ou évents 30 et sort de la seringue par le trou central de l'aiguille 24. Comme on le voit sur la figure 2, le surmoulage de l'aiguille 24 n'a pas pu s'encliqueter sur le piston parce que la distance séparant le piston du porte-aiguille est maintenue à une valeur suffisante par la bague 29. Il est à remarquer que dans cette position une étanchéité parfaite est assurée entre la face frontale du porte-aiguille et l'opercule 9 en matière élastomère donc compressible élastiquement.
3. L'usager place l'aiguille 24 soit dans un récipient, flacon, éprouvette, etc. où un liquide doit être prélevé, ou introduit cette aiguille dans la veine d'un patient, puis en déplaçant le piston 12-23 vers l'extrémité proximale de la seringue, il aspire le liquide ou le sang, à travers l'aiguille dans le cylindre 1 entre le porte-aiguille, définitivement fixé au cylindre, et le piston (figure 3). Arrivé en fin de course du piston ou ayant prélevé la quantité de liquide ou de sang voulu, l'usager retire l'aiguille du récipient ou de la veine du patient.
4. Ensuite, si l'on a prélevé du sang d'un patient, l'usager place l'aiguille dans une éprouvette ou si la seringue a été remplie d'un liquide, l'usager introduit l'aiguille dans la veine, le muscle, etc. d'un patient. L'usager déplace alors le piston en direction de l'extrémité distale de la seringue vidant le contenu de celle-ci soit dans une ou plusieurs éprouvettes, soit dans la veine, le muscle, etc. du patient. Arrivé en fin de course (figure 4), la bague 29 du porte-aiguille s'étant ramollie au contact du liquide ou du sang contenu dans la seringue, le piston se déplace jusqu'à ce que la partie conique 17 de la jupe bute contre le disque 25 du surmoulage de l'aiguille et que au moins un des crochets 27 des lamelles 26 de ce surmoulage vienne se crocheter sur la face 23 du crochet 20 du piston. Ce faisant, les secondes formations 32 du porte-aiguille ont été écartées par la portion tronconique 17 de la jupe du piston et lorsque l'usager déplace à nouveau le piston en direction de l'extrémité proximale de la seringue, celui-ci entraîne l'aiguille, qui se sépare du porte-aiguille, avec lui. Arrivé en fin de course (figure 5), l'aiguille 24 est entièrement rétractée dans le cylindre 1, son extrémité est située au-dessus du porte-aiguille définitivement fixé à la pièce de fermeture distale. L'opercule 9 en élastomère se referme, de sorte que le cylindre de la seringue est à nouveau étanche.
   En outre, du fait de la forme conique du crochet 20 du piston et de son décalage par rapport à l'axe du cylindre, l'aiguille s'incline sous l'action élastique des lamelles 26 de manière à former un angle par exemple de 5° à 7°, par rapport à l'axe du cylindre. Ceci interdit formellement toute possibilité de faire ressortir l'aiguille usagée du cylindre de la seringue. Pour encore augmenter ou assurer cet effet d'inclinaison de l'aiguille, il est possible de donner à la face inférieure de la jupe 17 du piston une inclinaison pour que cette face frontale soit par exemple parallèle à la face d'accrochage 23 du crochet 20. Ainsi, l'appui de la jupe 17 sur le disque 25 du surmoulage de l'aiguille force également à un décalage angulaire de l'aiguille en position rétractée.

Comme on le voit, pour l'usager l'utilisation de la seringue est très simple, il suffit de mouvements rectilignes entre le piston 14 et le cylindre 1. Tous les accouplements nécessaires fonctionnent automatiquement et sans que l'usager n 'ait à se préoccuper de la position angulaire relative des pièces à accoupler. En fin de course d'expulsion du liquide contenu dans la seringue, l'accouplement de l'aiguille au piston est automatique et irréversible, de sorte qu'une seconde utilisation de la seringue est impossible; en effet, pour reremplir la seringue, il faut rétracter le piston, ce qui entraîne automatiquement l'aiguille et la verrouille dans le cylindre.

Comme tous les éléments qui sont entrés en contact avec le sang ou un autre liquide et qui donc peuvent être contaminés sont en fin de processus enfermés de façon hermétique, la seringue peut être jetée sans autre précaution.

On remarque encore que pendant et après l'utilisation les deux mains de l'opérateur sont toujours du côté proximal donc non dangereux de la seringue, une main pour tenir le cylindre et l'autre pour tirer le piston qui entraîne l'aiguille à l'intérieur du cylindre. Les autres systèmes exigent qu'une des mains opère du côté distal de la seringue ou du collecteur afin de couvrir avec un capuchon l'aiguille souillée voire la dévisser du corps de la seringue, moment le plus dangereux à cause du risque de blessure et de contamination de l'utilisateur. La présente seringue est donc beaucoup plus sûre que les systèmes existants.

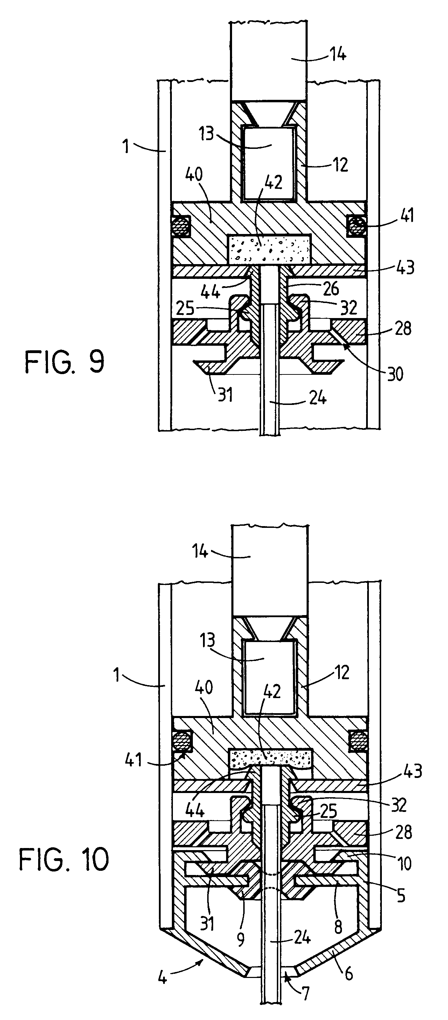

Dans la seconde forme d'exécution de la seringue illustrée aux figures 9 et 10, on retrouve pratiquement tous les éléments déjà décrits, seul l'accouplement entre le piston et l'aiguille, ainsi que la position de l'élément à dureté ou résistance modifiable sont différents.

Dans cette exécution, le piston coulissant dans le cylindre 1 comporte un corps 40 muni d'une gorge 41 recevant un joint d'étanchéité. La face supérieure de ce piston est munie de la partie 12 d'accouplement de la tige 14, tandis que la face distale du corps 40 comporte un logement renfermant un élément 42 en matière synthétique expansée ou en matière naturelle cellulaire capable de se ramollir au contact d'un liquide. Une plaque 43 fixée rigidement par collage, soudure, etc. contre la face distale du piston maintient l'élément poreux 42 en position et comporte un passage dont les bords sont biseautés allant en se rétrécissant en direction de l'élément 42.

Les languettes 26 du surmoulage de l'aiguille 24 comportent des crochets 44 destinés à pénétrer dans le logement du corps 40 lorsque l'élément 42 est ramolli par du liquide et à s'accoupler définitivement derrière la plaque 43 du piston (figure 10).

Le fonctionnement de cette seconde forme d'exécution de la seringue est en tout point similaire à celui de la première forme d'exécution décrite et présente donc les mêmes avantages. Dans cette exécution, pour provoquer lors du retrait de l'aiguille 24 dans le cylindre son déplacement angulaire interdisant de repousser l'aiguille hors du cylindre, il est possible de donner une longueur différente aux languettes 26 ou de donner une inclinaison à la face supérieure de la plaque 43.

De toute façon, même si l'aiguille n'est pas après usage de la seringue décalée angulairement par rapport à l'axe du cylindre, la seringue est inutilisable puisque l'aiguille est définitivement solidaire du piston et se déplace avec celui-ci. De plus son extrémité supérieure étant obturée par le piston, il est impossible par des mouvements alternés du piston de remplir ou vider le cylindre.

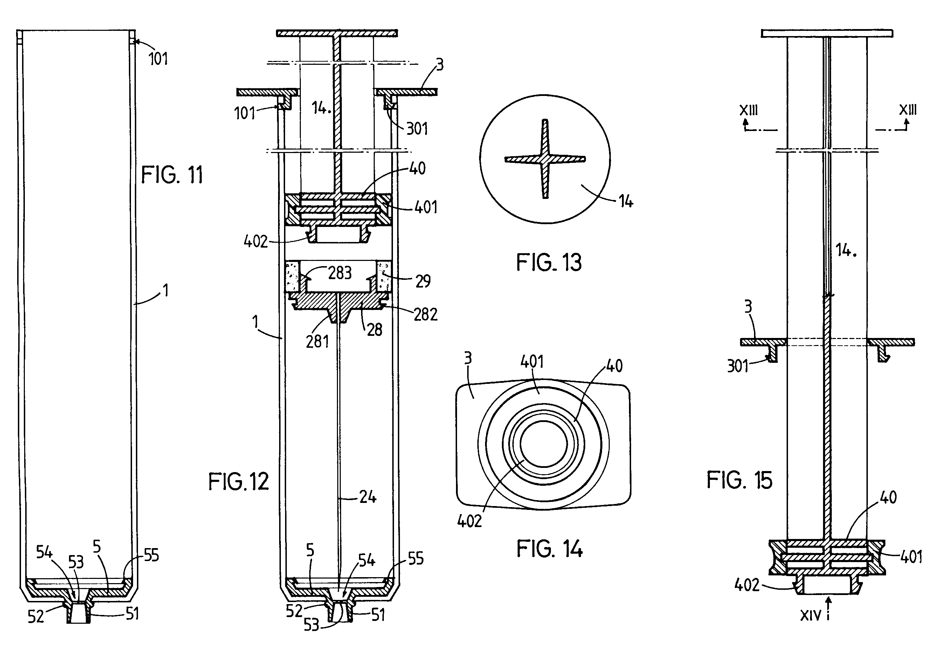

Dans la troisième forme d'exécution illustrée aux figures 11 à 20 l'aiguille 24 est portée, fixée solidairement au porte-aiguille 28. C'est ce porte-aiguille 28 qui présente des formations d'accouplement d'encliquetage non déverrouillables avec la pièce de fermeture distale 5 comme dans la première forme d'exécution. Ce porte-aiguille 28 comporte également sur son autre face, dirigée vers le piston 40, des formations d'encliquetage non déverrouillables coopérant avec le piston. Dans cette exécution l'élément à résistance variable 29 est une bague portée par/ou fixée sur la face supérieure, dirigée vers le piston, du porte-aiguille.

Cette troisième forme d'exécution est particulièrement avantageuse car elle comporte peu de pièces qui toutes peuvent être obtenues par injection et leur montage peut être automatisé.

La figure 11 illustre le cylindre 1 de la seringue obtenu par injection dans lequel vient se loger la pièce de fermeture distale 5 constituée en un élastomère relativement rigide. Cette pièce de fermeture distale comporte une extension tubulaire centrale 51 émergeant hors du cylindre 1 par un trou frontal de celui-ci. Cette pièce de fermeture distale 5 est maintenue en position de service par un bourrelet 52 qui ayant été forcé au travers du trou central du cylindre empêche toute séparation ultérieure de ces deux pièces. L'extension tubulaire centrale 51 est avant usage de la seringue obturée par une paroi ou membrane 53 destinée à être percée par l'aiguille 24 lorsque celle-ci est expulsée hors du cylindre 1.

Sur la figure 12, on voit que dans cette troisième forme d'exécution l'aiguille 24 est solidaire, fixée par surmoulage ou surinjection, d'un porte-aiguille 28. La face inférieure distale, de ce porte-aiguille 28 présente des formes correspondantes à la face interne, proximale, de la pièce de fermeture 5. Notamment une extension conique 281 et des formations d'encliquetage 282 correspondant à l'ouverture 54 et aux formations d'encliquetage 55 de la pièce de fermeture distale 5.

Les formations d'encliquetage 55, 282 permettent de réaliser un accouplement temporaire, suffisamment résistant pour planter l'aiguille 24 dans un muscle ou une veine d'un patient ou pour percer l'opercule d'un récipient, mais permettant un désaccouplement pour le retrait de l'aiguille 24 par le piston 40 à l'intérieur du cylindre en fin d'utilisation.

La face arrière ou proximale du porte-aiguille 28 est munie de formations d'encliquetage 283 disposées concentriquement et ménageant un espace recevant l'élément à résistance variable 29 qui se présente ici comme une bague ou un anneau.

Le piston 40 est venu d'une pièce d'injection avec sa tige 14 et comporte sur sa périphérie un joint 401 assurant l'étanchéité avec le cylindre 1. Ce piston comporte sur sa face distale libre des formations d'encliquetage 402 destinées à coopérer avec les formations d'encliquetage 283 du porte-aiguille 28 et à constituer avec celles-ci un accouplement non démontable et non deverrouillable une fois encliqueté.

L'extrémité arrière du corps ou cylindre 1 est obturée par un plateau 3 qui avant utilisation de la seringue est reliée par des pattes pouvant être brisées à la tige 14 du piston. Ces pattes constituent une garantie qui lorsqu'elles sont intactes indiquent que la seringue n'a pas été utilisée. Le plateau ou obturateur proximal 3 de la seringue est fixé au cylindre par encliquetage des crochets 301 dans des trous 101 pratiqués dans le cylindre 1.

Les figures 13,14 sont respectivement une coupe de la tige 14 du piston et une vue en bout de l'extrémité distale du piston.

La figure 15 est une vue du piston 40, de sa tige 14 et du plateau de fermeture proximal 3 avant assemblage avec le cylindre 1.

Lorsque la seringue est vierge elle se trouve à l'état illustré à la figure 12. Pour utiliser la seringue l'utilisateur enfonce le piston 40 à l'aide de sa tige 14 dans le cylindre 1, ce faisant il casse les parties reliant la tige 14 au plateau 3.

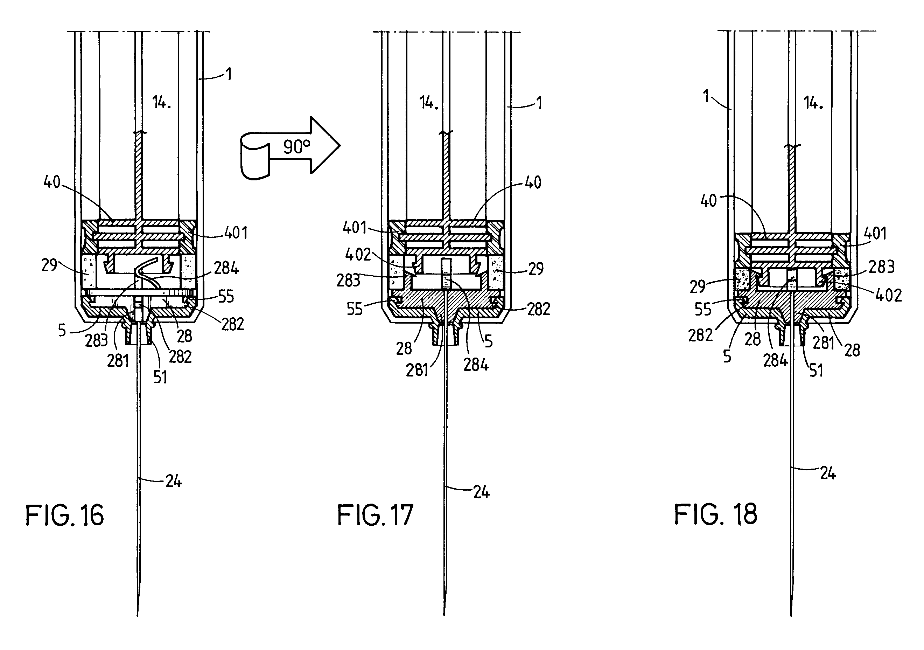

La face distale du piston 40 s'appuye sur la bague 29 et entraîne dans son déplacement le porte-aiguille 28 et l'aiguille 24. L'aiguille 24 perce la membrane élastomère 53 et le porte-aiguille est accouplé à la pièce de fermeture frontale 5 (figures 16 et 17). La bague 29 est sèche et donc rigide et ne peut pas s'écraser évitant ainsi tout accouplement du piston 40 au porte-aiguille 28. Dès cet instant, la seringue est opérationnelle, l'usager peut soit remplir le cylindre du sang d'un patient, soit d'un liquide contenu dans un flacon en déplaçant le piston dans le cylindre de façon à l'éloigner du porte-aiguille.

Comme dans les exécutions décrites précédemment, dès que la bague 29 est en contact avec un liquide elle perd sa rigidité et lorsque le piston 40 est à nouveau déplacé en direction du porte-aiguille il s'accouple définitivement à celui-ci (figure 18) en fin de course. Dès cet instant, la seringue est inutilisable puisque piston et aiguille ne peuvent plus que se déplacer simultanément et conjointement.

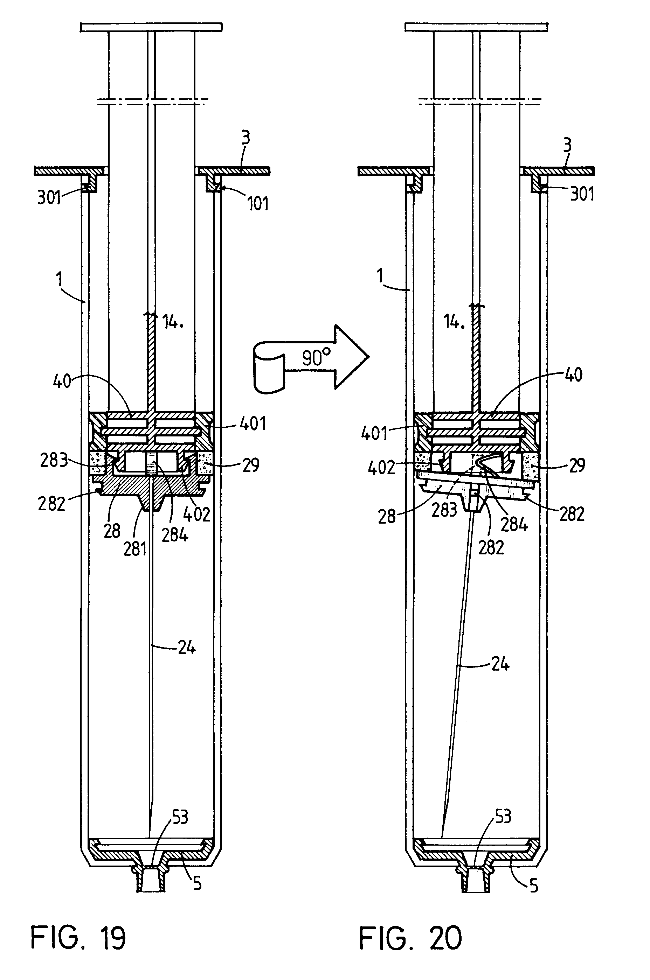

Ainsi, en retirant le piston par sa tige, le porte-aiguille 28 et l'aiguille 24 sont déplacés dans le cylindre, le porte-aiguille se désaccouplant de la pièce de fermeture proximale 5 ce qui provoque le retrait de l'aiguille dans le cylindre (fig. 19,20). Dès que l'aiguille 29 est entièrement dans le cylindre, la membrane élastomère 53 obture à nouveau l'extrémité frontale du cylindre et l'aiguille s'incline dans le cylindre sous l'effet de la lame ressort 284 faisant partie du porte-aiguille 28. Il est dès lors impossible de ressortir l'aiguille 24 du cylindre 1. Cette inclinaison de l'aiguille peut être provoquée par d'autres moyens, par exemple l'élasticité propre de la bague 29 ou la forme de la surface proximale du porte-aiguille.

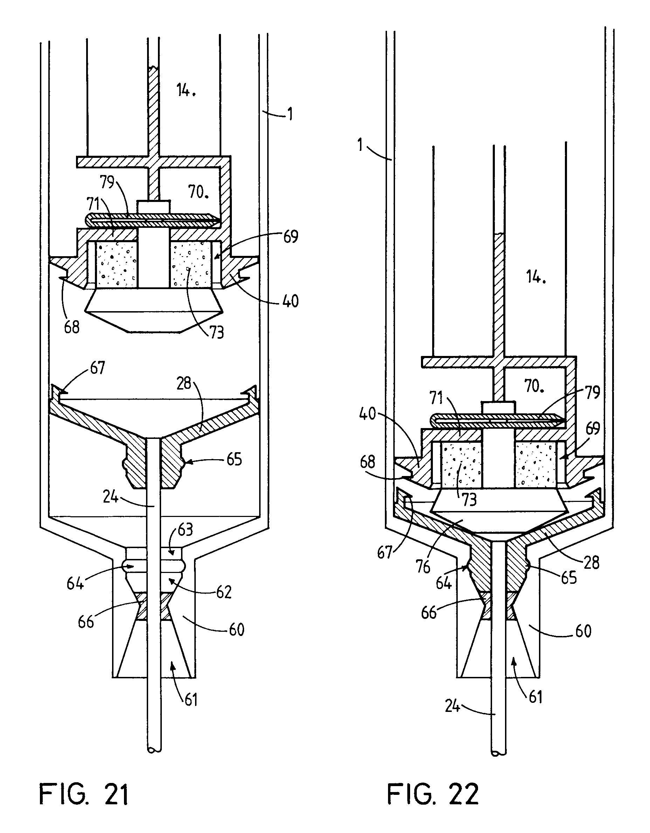

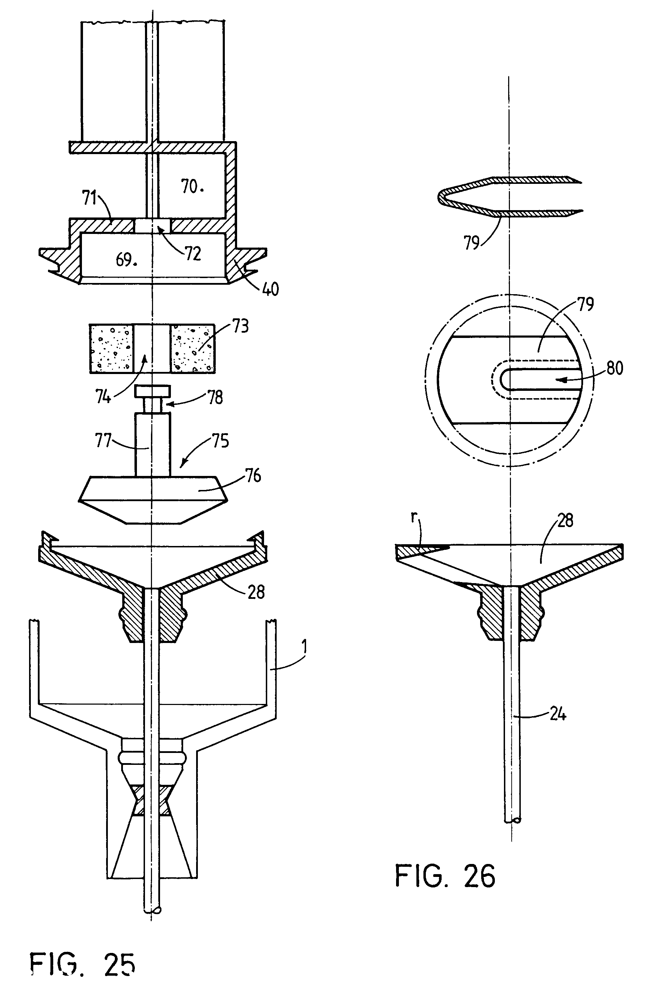

La quatrième forme d'exécution de la seringue selon l'invention, fig. 21 à 26, est toujours basée sur le même principe que décrit précédemment et son fonctionnement est en tout point similaire. Toutefois, cette réalisation comportant également peu de pièces permet encore une diminution du poids de matière plastique utilisée et une simplification des formes des pièces, et donc une réduction du coût de la seringue.

Dans cette forme d'exécution la seringue comporte également un cylindre creux 1 et un piston 14 dont les parties supérieures peuvent être réalisées comme dans la troisième forme d'exécution.

L'extrémité distale du cylindre comporte une extension cylindrique de plus faible diamètre 60 présentant un passage dont la forme est particulière. En effet, ce passage, reliant l'intérieur du cylindre 1 à l'extérieur de la seringue, comporte à son extrémité distale une portion tronconique 61 allant en se rétrécissant en direction du cylindre 1 suivit d'une portion tronçonnique inverse 62, allant en s'élargissant vers le cylindre 1. L'intersection de ces deux portions 61, 62 du passage définit un rétrécissement dudit passage. Ce passage comporte encore une troisième partie 63 reliant la partie tronconique 62 à l'intérieur du cylindre 1, partie 63 de forme générale cylindrique munie d'une gorge 64 de plus grand diamètre constituant l'une des parties de l'accouplement encliquetable et désencliquetable entre le porte-aiguille 28 et le cylindre 1. L'autre partie de cet accouplement, portée par le porte-aiguille 28, est constituée par un bourrelet circulaire 65 qui comporte l'extrémité distale du porte-aiguille 28 dont la forme et les dimensions correspondent à celles de la troisième partie 63 et au début de la seconde partie 62 du passage que comporte l'extension 60 du cylindre 1.

La zone du passage de l'extension 60 située de part et d'autre de son étranglement est remplie d'une masse élastomère 66 obturant hermétiquement le cylindre 1 tout en permettant le passage de l'aiguille et la refermeture hermétique du passage 61,62,63 l'aiguille ayant été rétractée dans le cylindre après usage.

L'extrémité proximale du porte-aiguille 28 présente une forme conique allant en s'évasant vers le piston et sa surface périphérique coulisse sans jeu dans ce cylindre 1. Cette extrémité proximale du porte-aiguille 28 comporte encore des fonctions d'encliquetage 67 coopérant avec des formations d'encliquetage 68 de l'extrémité distale du piston pour former un accouplement non déverrouillable une fois encliqueté.

Le piston coulisse sans jeu dans le cylindre 1 et comporte une cavité frontale 69 ainsi qu'un évidement 70 débouchant latéralement. La paroi 71 séparant la cavité frontale 69 de l'évidement 70 est munie d'un orifice 72.

Dans cette forme d'exécution de la seringue l'élément dont la résistance à la compression est plus faible à l'état humide ou mouillé qu'à l'état sec est constitué par un cylindre ou prisme 73 percé d'un passage central 74.

Cet élément 73 est fixé au piston à l'aide d'un arrêt 75 comprenant une tête 76 dont la partie distale est tronçonique et une tige 77 traversant l'élément 73 et la paroi 71 du piston 14. Cette tige 77 comporte une gorge 78 qui en position assemblée est située dans l'évidement 70 et reçoit un ressort à lame 79 présentant une entaille 80 donnant passage à la tige 77 dans sa partie de diamètre réduit par la gorge 78. Ainsi, l'élément à résistance variable 73 est maintenu en place serré par le ressort 79 entre la tête 76 de l'arrêt 75 et la paroi 71 du piston.

Il va de soi que les dimensions de ces différents éléments sont telles que lorsque l'élément à résistance variable 73 est à l'état sec et que le porte-aiguille 28 entre en contact avec la tête 76 de l'arrêt 75 porté par le piston (voir figure 22), il est possible d'encliqueter le porte-aiguille 28 avec le cylindre 1 par l'accouplement 64,65 mais la distance séparant les organes d'encliquetage 67,68 du porte-aiguille 28 et du piston 14 est trop grande pour que ces deux pièces puissent s'accoupler.

On peut ainsi pousser le porte-aiguille 28 à l'aide du piston pour faire sortir l'aiguille du cylindre en perforant l'élastomère et accoupler temporairement le porte-aiguille au cylindre (fig. 22).

En retirant ensuite le piston pour aspirer le liquide dans le cylindre, l'élément 73 se ramollit au contact du liquide et il est comprimé par l'action du ressort 79 et de l'arrêt 75 réduisant ainsi la distance entre la partie distale de l'arrêt 75 et les formations d'encliquetage 68 du piston.

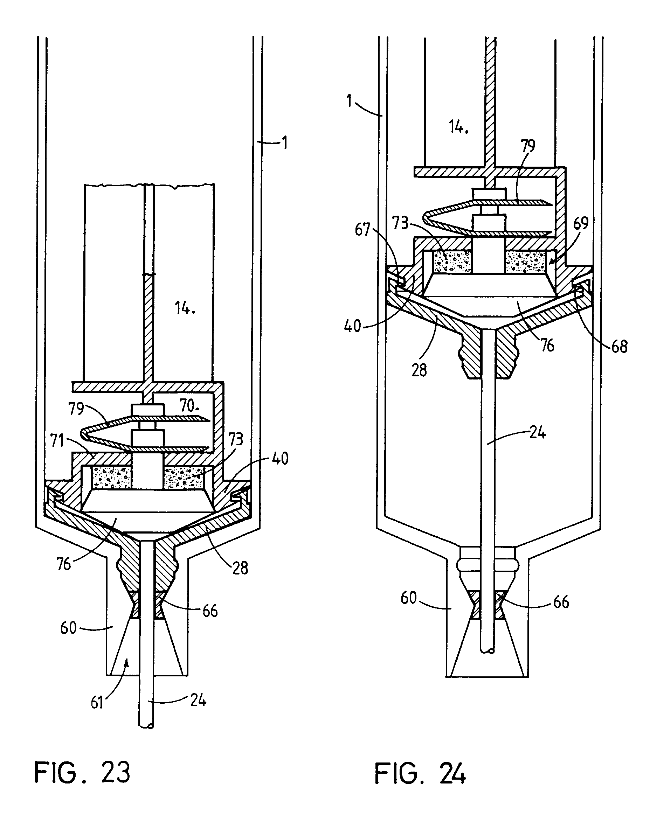

De cette façon, en fin d'injection d'un liquide à un patient (figure 23) ou de vider la seringue après un prélèvement, le piston s'accouple définitivement au porte-aiguille 28 à l'aide des formations 67,88 et lorsque le piston est retiré il entraîne le porte-aiguille de manière à rétracter, comme décrit précédemment, l'aiguille 24 dans le cylindre 1 (figure 24).

Dans cette forme d'exécution également, des moyens sont prévus pour qu'en fin de rétraction du piston, le porte-aiguille soit basculé par un ressort r par exemple pour incliner l'aiguille 24 par rapport à l'axe du cylindre et ainsi éviter qu'elle ne puisse être ressortie de celui-ci.

A part la question constructive et les formes des pièces particulièrement bien adaptées à une production en grande série, cette forme d'exécution présente l'avantage que l'élément compressible 73 est, lorsqu'il est humidifié par le liquide pénétrant dans le cylindre, comprimé par le ressort 79. Ceci permet de diminuer la force nécessaire en fin de course d'injection pour accoupler définitivement le piston au porte-aiguille.

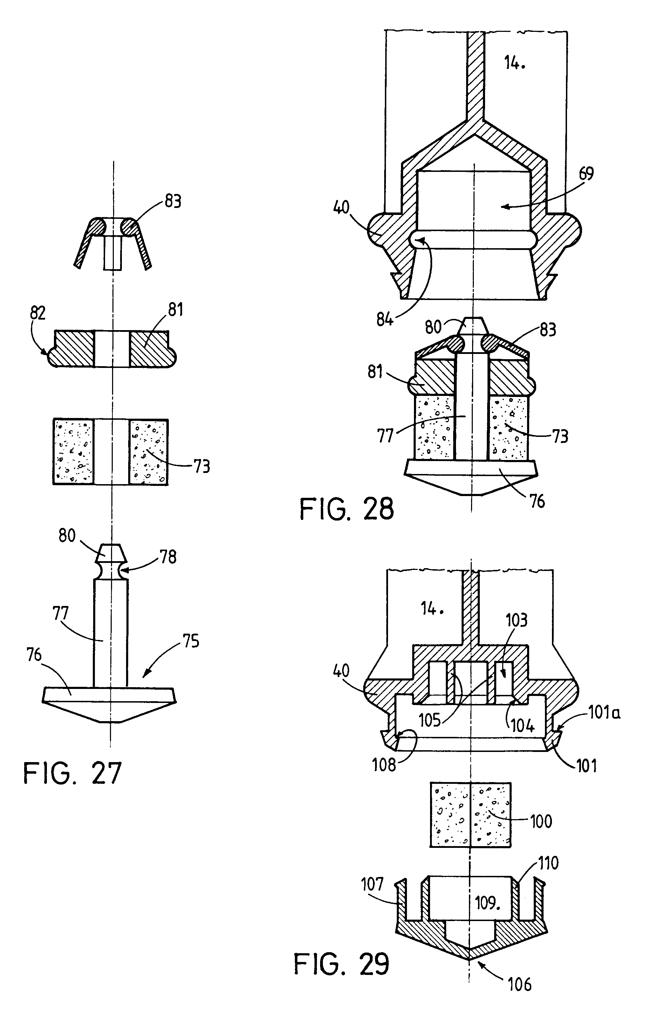

Les figures 27 et 28 illustrent une variante de cette quatrième forme d'exécution concernant la fixation de l'élément à résistance variable à l'état sec et humide 73 sur le piston.

Ici, cet élément 73 est enfilé sur la tige 77 de l'arrêt 75 et l'extrémité proximale de cette tige comporte une gorge 78 et une extrémité tronconique 80. Un disque 81 est enfilé sur cette tige 77 par dessus l'élément 73 et un ressort 83 à lamelles solides comprime le tout et est encliqueté par son orifice central dans la gorge 78 de la tige 77.

Ce disque 81 comporte un bourrelet circulaire 82 permettant d'accoupler l'ensemble arrêt 75, élément déformable 73, disque 81 et ressort 83 dans la cavité distale 69 du piston qui comporte une gorge circulaire 84 recevant le bourrelet 82 du disque 81.

L'avantage de cette variante réside dans le fait que l'ensemble formé de l'arrêt 75, de l'élément 73, du disque 81 et du ressort 83 peut être pré-monté et stocké. Le montage de la seringue en est d'autant simplifié.

La figure 29 illustre une variante de la fixation de l'élément compressible 100, ici de forme cubique, dont la résistance à la compression est plus grande à l'état sec qu'à l'état humide ou mouillé.

Dans cette variante, l'extrémité distale du piston comporte une paroi cylindrique 101 présentant un rebord externe 101a constituant la partie de son accouplement, non désencliquetable avec le porte-aiguille 28 (non illustré) qui comporte des organes d'accouplement correspondants. La face distale du piston comporte un logement 103 dont les bords sont chanfreinés 104, logement divisé par des parois 105 servant de butée à l'élément compressible 100.

Un bouchon 106 dont la face distale présente la même forme que celle de l'arrêt 75 de la forme d'exécution précédente mais qui présente un logement 109 recevant l'élé-ment déformable 100 et des crochets 107 permettant la fixation de ce bouchon sur un épaulement de retenue 108 que comporte la paroi cylindrique 101 du piston.

Ainsi, l'élément 100 peut être fixé à l'état sec, non comprimé, sur l'extrémité distale du piston.

Le logement 109 du bouchon 106 est délimité par une paroi 110 dont l'extrémité est biseautée et placée de manière à pouvoir entrer en contact avec les rebords 104 biseautés du logement 103 du piston lorsque l'élément déformable 100 est comprimé.

Ainsi à l'état mouillé et comprimé, l'élément 100 est entièrement emprisonné entre la tête du piston et le bouchon, par exemple de façon étanche.

Dans cette variante une pièce de plus a pu être supprimée et le montage de l'élément 100 sur le piston s'en trouve encore facilité.

Dans cette variante on peut également obtenir une compression automatique de l'élément compressible 100 en prévoyant un ressort en acier inoxydable tendant à rapprocher le bouchon 106 de la tête du piston. Un tel ressort peut présenter la forme d'une bague cylindrique par exemple en acier inox dont la paroi externe est en contact avec la surface interne de la paroi cylindrique 101 de la tête du piston. Cette bague est maintenue dans cette position par l'épaulement de retenue. Cette bague comporte plusieurs languettes s'étendant en direction du centre de la bague, languettes prenant appui contre la face frontale tronçonique du bouchon 106. Cette face frontale peut comporter des creusures à cet effet.

Ainsi les languettes tendent à rapprocher par leur élasticité propre le bouchon 106 de l'extrêmité distale du piston et donc à comprimer l'élément à résistance variable 100 dès que celui-ci est ramolli.

## Revendications

1. Seringue à usage médical comprenant un corps cylindrique, un piston déplaçable linéairement dans le corps, un porte-aiguille également déplaçable linéairement dans le corps et par rapport au piston et une aiguille, caractérisée par le fait que l'aiguille, avant utilisation de la seringue, est située, hermétiquement protégée, à l'intérieur du cylindre; par le fait qu'elle est portée par le porte-aiguille; par le fait que le corps de la seringue est obturé à son extrémité distale par un opercule élastomère perforable par l'aiguille; par le fait que le porte-aiguille et l'extrémité distale du corps comportent des formations d'accouplement par encliquetage; par le fait que l'extrémité proximale du porte-aiguille et le piston comportent des formations d'accouplement par encliquetage non déverrouillables une fois encliquetées; et par le fait que la distance minimale entre le piston et le porte-aiguille est déterminée par un élément dont la résistance à la compression est plus faible à l'état humide ou mouillé qu'à l'état sec.

2. Seringue selon la revendication 1, caractérisée par le fait que l'aiguille est montée sur le porte-aiguille par l'intermédiaire d'un accouplement du type bouton-pression permettant sa séparation du porte-aiguille; par le fait que le porte-aiguille et la pièce de fermeture distale comportent des formations d'accouplement par encliquetage non déverrouillables une fois encliquetées;

3. Seringue selon la revendication 1, caractérisée par le fait que lorsque l'élément à résistance variable est à l'état sec, la distance minimale entre l'extrémité proximale de l'aiguille et le piston est telle que cette aiguille ne puisse pas être accouplée au piston, mais que le porte-aiguille puisse être accouplé définitivement au cylindre maintenant l'aiguille en position de service hors du cylindre.

4. Seringue selon la revendication 3, caractérisée par le fait que lorsque l'élément à résistance variable est à l'état humide ou mouillé, un déplacement axial du piston par rapport à l'aiguille permet leur accouplement définitif par simple pression axiale, permettant ainsi le retrait complet de l'aiguille à l'intérieur du cylindre lors du retrait du piston.

5. Seringue selon la revendication 4, caractérisée par le fait que lorsque le porte-aiguille est accouplé, par simple pression axiale, avec la pièce de fermeture distale du corps cylindrique, l'opercule élastomère de cette pièce de fermeture assure l'étanchéité tant avec l'aiguille qu'avec le porte-aiguille.

6. Seringue selon l'une des revendications 1 à 5, caractérisée par le fait qu'avant usage le corps cylindrique est hermétiquement fermé et renferme l'aiguille, et est stérilisable.

7. Seringue selon l'une des revendications précédentes, caractérisée par le fait que l'accouplement indéconnectable entre le piston et l'aiguille est tel que lorsque l'aiguille est rétractée dans le corps cylindrique après usage, elle soit automatiquement décalée angulairement par rapport à l'axe du corps.

8. Seringue selon l'une des revendications précédentes, caractérisée par le fait que l'élément à résistance variable selon son état sec ou mouillé est constitué par une bague portée par le porte-aiguille.

9. Seringue selon l'une des revendications 1 à 8, caractérisée par le fait que l'élément à résistance variable selon son état sec ou mouillé est porté par la face avant du piston.

10. Seringue selon l'une des revendications précédentes, caractérisée par le fait que le corps cylindrique est constitué d'une section de tube extrudé, d'un couvercle fixé sur sa face proximale et muni d'une ouverture laissant passer une partie arrière du piston et d'une pièce de fermeture distale munie d'un opercule perforable par l'aiguille.

11. Seringue selon la revendication 10, caractérisée par le fait que la pièce de fermeture distale comporte un logement au-delà de l'opercule destiné à récupérer et isoler toute souillure de la surface externe de l'aiguille lors de sa rétraction dans le corps cylindrique.

12. Seringue selon l'une des revendications précédentes, caractérisée par le fait que l'élément à résistance variable suivant son état sec ou mouillé est constitué par une masse de matière synthétique ou naturelle.

13. Seringue selon la revendication 11, caractérisée par le fait que le logement de la pièce de fermeture distale comporte une portion, située dans l'axe de la seringue, pouvant être détachée pour donner passage à l'aiguille.

14. Seringue selon la revendication 1 ou l'une des revendications 3 à 13, caractérisée par le fait que l'aiguille est solidaire du porte-aiguille.

15. Seringue selon l'une des revendications précédentes, caractérisée par le fait que l'extrémité distale du corps comporte une pièce de fermeture comportant l'opercule élastomère; et par le fait que les formations d'accouplement de l'extrémité distale du corps sont portées par ladite pièce de fermeture.

16. Seringue selon l'une des revendications 1,2,3,4,5,6,7 ou 9, caractérisée par le fait que l'élément à résistance variable suivant son état sec ou mouillé est fixé dans la partie frontale du piston et soumis à l'action d'un ressort tendant à le comprimer.

17. Seringue selon la revendication 16, caractérisée par le fait que cet élément à résistance variable fait partie d'un ensemble fixé de façon amovible dans la partie frontale du piston.

18. Seringue selon l'une des revendications 1 à 7 ou 9, caractérisée par le fait que l'élément à résistance variable est fixé sur la face distale du piston à l'aide d'un bouchon, cet élément à résistance variable étant à l'état comprimé de faible volume, enfermé de façon étanche dans un espace fermé entre le piston et le bouchon

19. Seringue selon la revendication 18, caractérisée par le fait qu'un ressort tend à déplacer le bouchon en direction de la tête du piston provoquant la compression de l'élément à résistance variable entre ces deux éléments.
